# DEMANDE DE BREVET EUROPEEN

(11) **EP 1 121 952 A1**
(43) Date de publication de la demande: **08.08.2001**
(21) Numéro de dépôt: 01400083.0
(22) Date de dépôt: 12.01.2001
(51) Int. Cl.: A61M 16/01

(54) **Ventilateur d'anesthésie avec contrôle automatique du mode de ventilation selectionné**

(30) Priorité: 07.02.2000 FR 0001480
(71) Demandeur: TAEMA, F-92182 Antony Cédex (FR)
(72) Inventeur: Kitten, Sébastien, 91160 Saulx les Chartreux (FR)
(74) Mandataire: Pittis, Olivier

(57) **Abrégé**

L'invention se rapporte à un appareil d'anesthésie du type à plusieurs modes de ventilation comportant un circuit principal (1) de gaz formé d'une branche inspiratoire (1a) et d'une branche expiratoire (1b) ; un organe d'accumulation (4) de gaz dont le volume interne varie et communique avec le circuit principal (1) et des moyens à valve de décharge (6) à niveau de pression de consigne réglable agencés sur le circuit principal (1). En outre, des moyens de détection (11, 12, 13, 14) permettant de déterminer une information du gaz entrant et/ou sortant de l'organe d'accumulation (4), des moyens de traitement (10) d'information permettent de traiter ladite information pour en déduire une information de mode de ventilation, et des moyens de commande (15) agissent sur les moyens à valve de décharge (6) pour ajuster automatiquement le niveau de pression de consigne desdits moyens à valve de décharge (6) en fonction de l'information de mode de ventilation délivrée par les moyens de traitement (10).

## Description

La présente invention se rapporte au domaine de l'anesthésie inhalatoire et, plus particulièrement, à un appareil d'anesthésie inhalatoire comprenant un circuit de gaz équipé de moyens permettant de surveiller, de préférence en permanence, les mouvements ou flux de gaz entrant et sortant d'un réservoir d'accumulation de gaz, tel un ballon-réservoir équipant ledit circuit de gaz.

Les appareils d'anesthésie comportent classiquement un système fermé de canalisations de gaz ou analogues, encore appelé circuit principal.

Un circuit principal est destiné, d'une part, à amener jusqu'aux voies aériennes supérieures du patient des gaz anesthésiques frais issus d'une source de gaz anesthésiques frais et, d'autre part, de récupérer les gaz exhalés par le patient, de permettre une élimination du dioxyde de carbone contenu dans ces gaz exhalés par le patient, de permettre un recyclage et un mélange de ces gaz exhalés purifiés avec les gaz anesthésiques frais avant leur renvoi vers les voies aériennes supérieures du patient.

Un tel circuit est donc un système fermé fonctionnant en boucle et qui permet de minimiser la quantité de gaz anesthésique frais utilisée pour anesthésier le patient.

En général, le circuit en boucle comporte un organe d'accumulation, tel un ballon-réservoir de gaz, destiné à stocker temporairement au moins une partie des gaz expirés par le patient et qui ont été purifiés par élimination d'au moins la majeure partie du dioxyde de carbone qu'ils contenaient.

Les gaz expirés et purifiés sont réutilisés et renvoyés vers le patient durant les phases inspiratoires subséquentes, en étant éventuellement additionnés de gaz anesthésiques frais.

De tels dispositifs ont déjà été décrits à maintes reprises dans l'art antérieur. Pour plus de détails, on peut se reporter notamment aux documents EP-A-643978, US-A-3687137, EP-A-292615 ou EP-A-266964.

Actuellement, les appareils d'anesthésie inhalatoires existants fonctionnent selon trois modes primaires de ventilation, à savoir le mode spontané, le mode manuel et le mode contrôlé.

En mode spontané, le patient doit pouvoir puiser de façon autonome la quantité de gaz respiratoire dont il a besoin au sein de l'organe d'accumulation des gaz expirés, par exemple un ballon-réservoir de gaz.

En mode manuel, c'est le praticien qui commande l'expulsion du gaz contenu dans l'organe d'accumulation vers le patient en exerçant une pression sur la paroi souple de l'organe d'accumulation.

En mode contrôlé, encore appelé mode mécanique, la ventilation du patient est complètement automatisée, c'est-à-dire générée par le ventilateur qui assure et gère la circulation du gaz entre le patient et l'organe d'accumulation du gaz, et vice versa.

Cependant, le passage d'un mode de ventilation mécanique ou spontané vers le mode de ventilation manuel ne peut être effectué actuellement que par le biais d'une commutation manuelle d'une soupape de décharge par le praticien, c'est-à-dire le médecin ou analogue.

Or, on comprend aisément que se pose un réel problème de sécurité.

En effet, l'omission de la commutation du mode manuel vers le mode spontané, par exemple, force le patient à lutter contre des fortes pression de gaz, en général des pressions de 10 à 90 h Pa, lesquelles peuvent engendrer un barotraumatisme chez le patient du fait de la montée en pression de l'ensemble du circuit de ventilation et, par conséquent, des voies aériennes supérieures et des poumons dudit patient.

Le but de la présente invention est donc de proposer un appareil d'anesthésie inhalatoire et un procédé de commande d'un tel appareil permettant de résoudre les problèmes précités et donc de minimiser les risques pour le patient.

En d'autres termes, la présente invention vise à réduire le fonctionnement d'un appareil d'anesthésie à seulement deux modes de ventilation, à savoir un mode assisté et un mode contrôlé, grâce à une reconnaissance automatique par l'appareil du passage entre notamment le mode spontané et le mode manuel, et à une reproduction automatique des commutations préalablement déclenchées manuellement par le praticien.

La présente invention permet donc de simplifier l'utilisation des dispositifs ou machines d'anesthésie et d'augmenter la sécurité pour le patient.

La présente invention concerne alors un appareil d'anesthésie du type à plusieurs modes de ventilation comportant au moins :
- un circuit principal de gaz formé d'au moins une branche inspiratoire et d'au moins une branche expiratoire,
- un organe d'accumulation de gaz dont le volume interne est susceptible de varier et communiquant avec ledit circuit principal de gaz,
- des moyens à valve de décharge à niveau de pression de consigne réglable agencés sur ledit circuit principal de gaz,
- des moyens de détection permettant de déterminer au moins une information du gaz entrant et/ou sortant de l'organe d'accumulation,
- des moyens de traitement d'information permettant de traiter ladite au moins une information du gaz entrant et/ou sortant pour en déduire au moins une information de mode de ventilation, et
- des moyens de commande agissant sur les moyens à valve de décharge pour ajuster automatiquement le niveau de pression de consigne desdits moyens à valve de décharge en fonction d'au moins l'information de mode de ventilation délivrée par lesdits moyens de traitement d'information.

Selon le cas, l'appareil selon l'invention peut comprendre l'une ou plusieurs des caractéristiques suivantes :
- lesdits moyens de détection sont agencés sur ou à proximité de l'organe d'accumulation de gaz ;
- lesdits moyens de détection sont choisis parmi :
   - les capteurs de débit de gaz entrant et/ou sortant de l'organe d'accumulation de gaz,
   - les capteurs de pression de gaz entrant et/ou sortant de l'organe d'accumulation de gaz,
   - les commutateurs agencés sur ou à proximité du raccord de l'organe d'accumulation et actionnés directement par l'utilisateur lorsqu'il souhaite commander lesdits moyens à valve de décharge,
   - les transmetteurs mécaniques permettant de traduire une variation de pression dans l'organe d'accumulation en au moins une information mécanique ou électrique, et/ou
   - les dispositifs d'analyse des variations du volume et/ou de la forme et/ou de la pression de l'organe d'accumulation de gaz,
- l'organe d'accumulation de gaz est un ballon-réservoir ;
- les moyens de traitement d'information comprennent au moins un microprocesseur apte à traiter les informations électroniques transmises par les moyens de détection pour en déduire au moins une information du mode de ventilation, c'est-à-dire que le microprocesseur permet la prise en compte du mode de ventilation imposé par l'opérateur et assure les commandes électroniques des moyens de ventilation ;
- les moyens de commande agissent sur les moyens à valve de décharge pour diminuer automatiquement le niveau de pression de consigne desdits moyens à valve de décharge lorsque l'information de mode de ventilation délivrée par les moyens de traitement d'information correspond à une information de mode de ventilation spontanée ;
- les moyens de commande agissent sur les moyens à valve de décharge pour augmenter automatiquement le niveau de pression de consigne desdits moyens à valve de décharge lorsque l'information de mode de ventilation délivrée par les moyens de traitement d'information correspond à une information de mode de ventilation manuelle ;
- l'organe d'accumulation de gaz et/ou les moyens à valve de décharge sont agencés sur la branche expiratoire ou sur la branche inspiratoire ;
- des moyens de détection sont agencés sur une ligne de liaison reliant l'organe d'accumulation de gaz au circuit principal de gaz.

L'invention concerne, en outre, un procédé de commande d'un appareil d'anesthésie du type à plusieurs modes de ventilation, en particulier, selon l'invention, comprenant au moins un circuit principal de gaz formé d'au moins une branche inspiratoire et d'au moins une branche expiratoire, au moins un organe d'accumulation de gaz communiquant avec le circuit principal de gaz, et des moyens à valve de décharge à niveau de pression de consigne réglable agencés sur ledit circuit principal de gaz, dans lequel :
(a) on détermine au moins une information du gaz entrant et/ou sortant de l'organe d'accumulation,
(b) on traite ladite au moins une information du gaz entrant et/ou sortant,
(c) on déduit de l'étape (b) au moins une information de mode de ventilation, et
(d) on ajuste automatiquement le niveau de pression de consigne en agissant sur lesdits moyens à valve de décharge en fonction d'au moins l'information de mode de ventilation obtenue à l'étape (c).

Selon le cas, le procédé selon l'invention peut comprendre l'une ou plusieurs des caractéristiques suivantes :
- on ajuste automatiquement le niveau de pression de consigne à une valeur de pression de consigne inférieure ou égale à 5 hPa, de préférence inférieure ou égale à 2 hPa, lorsque l'information de mode de ventilation correspond à une information de mode de ventilation spontané ;
- on ajuste automatiquement le niveau de pression de consigne à une valeur de pression de consigne supérieure ou égale à 5 hPa, de préférence comprise entre 10 et 90 hPa, lorsque l'information de mode de ventilation correspond à une information de mode de ventilation manuelle ;
- à l'étape (a), on détermine au moins une information du gaz entrant et/ou sortant de l'organe d'accumulation choisie parmi la pression du gaz entrant et/ou sortant de l'organe d'accumulation, le débit du gaz entrant et/ou sortant de l'organe d'accumulation et la pression du gaz à l'intérieur de l'organe d'accumulation et/ou une information transmise par un commutateur agencé sur ou à proximité du raccord de l'organe d'accumulation.

La présente invention va maintenant être décrite plus en détail, en référence aux figures annexées, données à titre illustratif mais non limitatif, à savoir :
- la figure 1 représente le schéma du principe de fonctionnement d'un appareil d'anesthésie en mode spontané durant une phase inspiratoire du patient ;
- la figure 2 est analogue à la figure 1 mais schématise le mode spontané durant une phase expiratoire du patient ;
- la figure 3 représente, quant à elle, le principe de fonctionnement de l'appareil d'anesthésie en mode manuel durant une phase inspiratoire ; et
- la figure 4 représente le schéma du principe de fonctionnement d'un appareil d'anesthésie selon la présente invention.

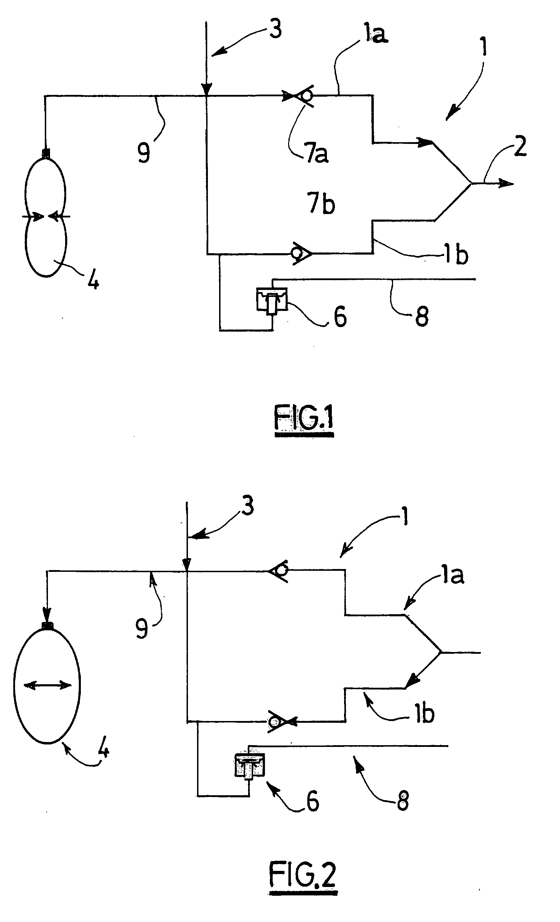

Sur les figures 1 à 3 est représenté un schéma d'un appareil d'anesthésie comportant un circuit principal 1 de gaz composé d'une branche inspiratoire la et d'une branche expiratoire 1b, formant un circuit en boucle ou circuit fermé.

La partie aval de la branche inspiratoire la est reliée aux voies aériennes supérieures d'un patient (non représenté) par l'intermédiaire d'un moyen de raccordement de sortie 2 ou embout de raccordement, par exemple un masque respiratoire ou une sonde d'intubation, permettant de distribuer un gaz anesthésique aux voies respiratoires supérieures dudit patient.

En outre, la branche inspiratoire la est reliée par son extrémité amont 3 à une source de gaz anesthésiques ou gaz « frais » (non représenté.), par exemple un mélange gazeux contenant de l'oxygène, de l'azote et des produits halogénés.

Par ailleurs, le circuit fermé 1 comporte également une branche expiratoire 1b dont l'extrémité amont se raccorde à l'extrémité aval de la branche inspiratoire la au niveau de l'embout de raccordement 2, de manière à pouvoir recueillir les gaz expirés par le patient, et dont l'extrémité aval se raccorde à l'extrémité amont 3 de la branche inspiratoire 1a, de manière à constituer le circuit fermé 1.

Par ailleurs, le circuit principal 1 comporte un ballon-réservoir 4.

La branche expiratoire 1b comporte habituellement un dispositif de purification (non représenté) permettant d'éliminer au moins une partie du dioxyde de carbone (CO₂) contenu dans les gaz expirés par le patient, par exemple une cartouche d'adsorbant.

La branche expiratoire 1b comporte également un échappement 8 vers l'atmosphère muni d'une soupape de décharge 6 et destiné à pallier toute surpression dans le circuit principal 1 due notamment à l'alimentation permanente du circuit principal en gaz frais, le trop plein étant évacué par la valve de décharge 6.

En outre, des clapets anti-retour 7a et 7b sont aménagés sur lesdites branches inspiratoire la et expiratoire 1b, respectivement.

L'organe d'accumulation 4, ici un ballon-réservoir, à volume interne variable est relié pneumatiquement au circuit principal 1 par le biais d'une ligne 9 de liaison.

Comme on peut le voir sur la figure 1, lorsque le patient inspire spontanément, le gaz contenu dans l'organe d'accumulation 4 sort dudit organe d'accumulation 4 et est acheminé jusqu'au patient par la ligne 9 et la branche inspiratoire la, en étant délivré au patient par le biais de l'embout 2 de raccordement.

Durant la phase inspiratoire, en mode spontané, la pression de gaz dans le circuit est généralement de l'ordre de 2 h Pa environ.

A l'inverse, ainsi que montré sur la figure 2, durant la phase expiratoire, le patient expire spontanément des gas expirés riches en CO₂, lesquels sont d'abord purifiés par élimination d'au moins une partie du CO₂ qu'ils contiennent, puis renvoyés, via les lignes 1b et 9, vers le ballon-réservoir 4 où ils sont stockés jusqu'à la phase inspiratoire suivante.

En général, durant la phase expiratoire, en mode spontané, la pression de gaz dans le circuit est aussi de l'ordre de 2 h Pa environ.

En d'autres termes, en mode spontané, que ce soit en phase respiratoire (fig. 1) ou en phase expiratoire (fig. 2), la pression de commande de la soupape 6 de décharge est réglée à 2 h Pa environ.

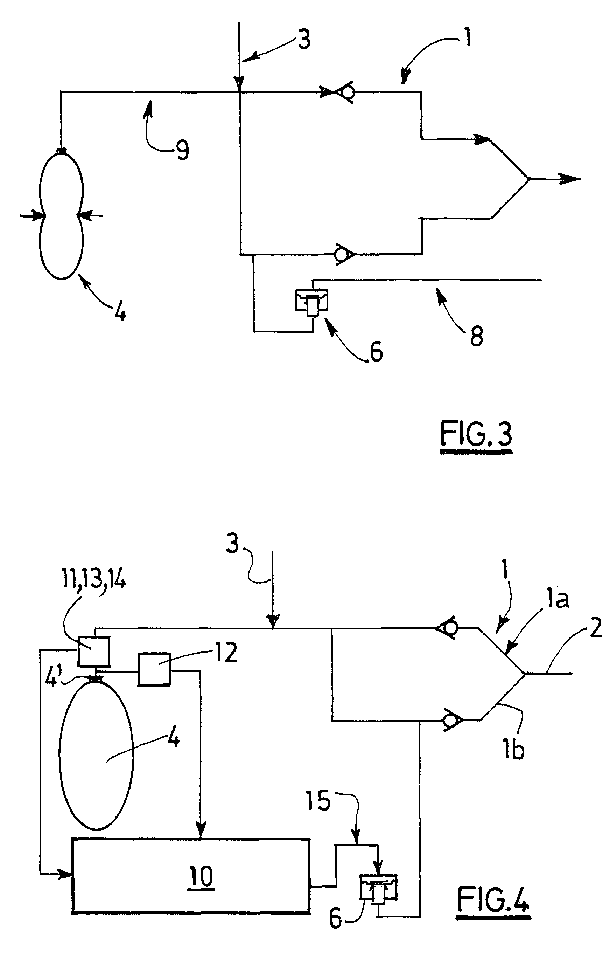

Par contre, ainsi que schématisé sur la figure 3, en mode manuel, durant les phase inspiratoires, il est nécessaire que le praticien exerce lui-même une pression sur le ballon-réservoir 4 pour en expulser, via les lignes 9 et la, le gaz accumulé et l'envoyer vers le patient.

En effet, les patients devant être ventilé manuellement sont ceux pour lesquels la profondeur d'anesthésie est telle qu'ils n'ont plus leur automatisme ventilatoire/respiratoire, c'est-à-dire que ceux-ci ne peuvent pas puiser par eux-mêmes le gaz stocké dans le ballon-réservoir 4.

En mode manuel, la pression de gaz du circuit de ventilation doit être donc réglée par le praticien à une valeur habituellement comprise entre 10 h Pa et 90 h Pa, de manière à ce que la pression manuelle que le praticien exerce sur le ballon-réservoir 4 pour en faire sortir le gaz n'endommage pas les poumons du patient tout en n'induisant pas pour autant un échappement intempestif de l'ensemble du gaz par la soupape 6 de décharge, laquelle est moins résistive que le patient.

Pour éviter ce problème, la pression de commande de ladite soupape 6 de décharge est fixée à une valeur désirée comprise entre 10 et 90 h Pa.

Dans les appareils actuels, durant la phase expiratoire, en mode manuel, la pression de commande ne varie pas.

Or, du point de vue de la sécurité, on comprend aisément que l'omission de la commutation du mode manuel vers le mode spontané oblige le patient à forcer sa respiration et à lutter contre les fortes pressions du mode manuel (10 à 90 h Pa), ce qui peut créer, dans certains cas, un barotraumatisme de par la montée en pression de l'ensemble du circuit du ventilateur et donc des poumons du patient.

Pour pallier ce problème et augmenter la sécurité du patient, la présente invention propose un appareil d'anesthésie dont le principe de fonctionnement est schématisé sur la figure 4.

En effet, pour éviter que le patient soit soumis à une surpression due à l'omission de la commutation manuelle par le praticien, lors du passage d'un mode de ventilation manuelle à un mode de ventilation spontanée, le ventilateur d'anesthésie selon l'invention est muni de moyens de détection, c'est-à-dire de capteurs 11 et 12, reliés ou situés sur ou à proximité immédiate du ballon-réservoir 4, par exemple sur la ligne 9 de liaison ou le raccord de connexion 4' reliant ladite ligne 9 de liaison audit ballon-réservoir 4.

Ces capteurs 11 et 12 permettent de déterminer un ou plusieurs paramètres ou informations représentatifs des mouvements de gaz entrant dans et/ou sortant du ballon-réservoir 4.

Après détermination de ces paramètres, ceux-ci sont transmis à des moyens de traitement 10 des données, telle une unité de traitement (CPU : Control Process Unit), où ces paramètres sont traités pour permettre à l'appareil de sélectionner, de manière fiable, le mode de ventilation à observer, selon que le mouvement de gaz (sortie de gaz) en fonction résulte d'un appel respiratoire spontané du patient ou, le cas échéant, d'une manoeuvre du praticien par pression manuelle sur ledit ballon- réservoir.

En fait, le fonctionnement est basé sur une détermination du mode d'insufflation manuel, à partir d'une ou plusieurs informations issues, par exemple de la mesure de pression de gaz au moyen d'un capteur de pression 12, de la mesure de débit de gaz au moyen d'un capteur de débit 11, de la mesure d'une composante du ballon-réservoir 4 lui-même (déformation, ...), de l'actionnement par le praticien d'un commutateur 13 situé sur ou à proximité du ballon-réservoir, ou encore de la retranscription par voie mécanique 14 de la pression régnant à l'intérieur dudit ballon-réservoir 4.

Sur la figure 4, le ventilateur représenté comprend un capteur de pression 12 et un capteur de débit 11 dont les prises de mesure sont situées à proximité immédiate de l'orifice de sortie du ballon-réservoir 4, sur la ligne 9.

En outre, les moyens 10 de traitement des données peuvent être un système à microprocesseur possédant une chaîne d'acquisition analogique lui permettant de convertir les mesures de pression 12 et/ou de débit 11 en valeurs numériques qui, après filtrage et correction numérique, peuvent être comparées à des valeurs seuil permettant de détecter si l'insufflation est imposée par une action manuelle de l'opérateur sur le ballon-réservoir 4 ou si elle résulte d'une aspiration par le patient.

Par ailleurs, les moyens de traitement 10 peuvent, à partir des mesures de pression 12 et/ou de débit 11, déterminer les caractéristiques du ballon-réservoir 4, telles que son élasticité et ses variations de volume, et déterminer, à partir de là, si la ventilation est manuelle ou spontanée.

Dans le cas où un commutateur 13 ou un transmetteur mécanique 14 sont placés à proximité immédiate du raccord du ballon-réservoir 4, ceux-ci transmettent directement aux moyens de traitement 10, l'information d'une action manuelle de l'opérateur sur le ballon-réservoir 4.

## Revendications

1. Appareil d'anesthésie du type à plusieurs modes de ventilation comportant au moins :
- un circuit principal (1) de gaz formé d'au moins une branche inspiratoire (1a) et d'au moins une branche expiratoire (1b),
- un organe d'accumulation (4) de gaz dont le volume interne est susceptible de varier et communiquant avec ledit circuit principal (1) de gaz,
- des moyens à valve de décharge (6) à niveau de pression de consigne réglable agencés sur ledit circuit principal (1) de gaz,
- des moyens de détection (11, 12, 13, 14) permettant de déterminer au moins une information du gaz entrant et/ou sortant de l'organe d'accumulation (4),
- des moyens de traitement (10) d'information permettant de traiter ladite au moins une information du gaz entrant et/ou sortant pour en déduire au moins une information de mode de ventilation, et
- des moyens de commande (15) agissant sur les moyens à valve de décharge (6) pour ajuster automatiquement le niveau de pression de consigne desdits moyens à valve de décharge (6) en fonction d'au moins l'information de mode de ventilation délivrée par lesdits moyens de traitement (10) d'information.

2. Appareil selon la revendication 1, caractérisé en ce que lesdits moyens de détection (11, 12, 13, 14) sont agencés sur ou à proximité de l'organe d'accumulation (4) de gaz.

3. Appareil selon l'une des revendications 1 ou 2, caractérisé en ce que lesdits moyens de détection (11, 12, 13, 14) sont choisis parmi :
- les capteurs (11) de débit de gaz entrant et/ou sortant de l'organe d'accumulation (4) de gaz,
- les capteurs (12) de pression de gaz entrant et/ou sortant de l'organe d'accumulation (4) de gaz,
- les commutateurs (13) agencés sur ou à proximité du raccord de l'organe d'accumulation (4) et actionnés directement par l'utilisateur lorsqu'il souhaite commander lesdits moyens à valve de décharge (6),
- les transmetteurs mécaniques (14) permettant de traduire une variation de pression dans l'organe d'accumulation (4) en au moins une information mécanique ou électrique, et/ou
- les dispositifs d'analyse des variations du volume et/ou de la forme et/ou de la pression de l'organe d'accumulation (4) de gaz.

4. Appareil selon l'une des revendications 1 à 3, caractérisé en ce que l'organe d'accumulation (4) de gaz est un ballon-réservoir.

5. Appareil selon la revendication 1, caractérisé en ce que les moyens de traitement (10) d'information comprennent au moins un microprocesseur.

6. Appareil selon l'une des revendications 1 ou 5, caractérisé en ce que les moyens de commande (14) agissent sur les moyens à valve de décharge (6) pour diminuer automatiquement le niveau de pression de consigne desdits moyens à valve de décharge (6) lorsque l'information de mode de ventilation délivrée par les moyens de traitement (10) d'information correspond à une information de mode de ventilation spontanée.

7. Appareil selon l'une des revendications 1 ou 5, caractérisé en ce que les moyens de commande (14) agissent sur les moyens à valve de décharge (6) pour augmenter automatiquement le niveau de pression de consigne desdits moyens à valve de décharge (6) lorsque l'information de mode de ventilation délivrée par les moyens de traitement (10) d'information correspond à une information de mode de ventilation manuelle.

8. Appareil selon l'une des revendications 1 à 7, caractérisé en ce que l'organe d'accumulation (4) de gaz et/ou les moyens à valve de décharge (6) sont agencés sur la branche expiratoire (1b) ou sur la branche inspiratoire (1a).

9. Appareil selon l'une des revendications 1 à 7, caractérisé en ce que des moyens de détection (11, 12) sont agencés sur une ligne de liaison (9) reliant l'organe d'accumulation (4) de gaz au circuit principal (1) de gaz.

10. Procédé de commande d'un appareil d'anesthésie du type à plusieurs modes de ventilation, en particulier selon l'une des revendications 1 à 9, comprenant au moins un circuit principal (1) de gaz formé d'au moins une branche inspiratoire (1a) et d'au moins une branche expiratoire (1b), au moins un organe d'accumulation (4) de gaz communiquant avec le circuit principal (1) de gaz, et des moyens à valve de décharge (6) à niveau de pression de consigne réglable agencés sur ledit circuit principal (1) de gaz, dans lequel :
(a) on détermine au moins une information du gaz entrant et/ou sortant de l'organe d'accumulation (4),
(b) on traite ladite au moins une information du gaz entrant et/ou sortant,
(c) on déduit de l'étape (b) au moins une information de mode de ventilation, et
(d) on ajuste automatiquement le niveau de pression de consigne en agissant sur lesdits moyens à valve de décharge (6) en fonction d'au moins l'information de mode de ventilation obtenue à l'étape (c).

11. Procédé selon la revendication 10, caractérisé en ce qu'on ajuste automatiquement le niveau de pression de consigne à une valeur de pression de consigne inférieure ou égale à 5 hPa, de préférence inférieure ou égale à 2 hPa, lorsque l'information de mode de ventilation correspond à une information de mode de ventilation spontané.

12. Procédé selon la revendication 10, caractérisé en ce qu'on ajuste automatiquement le niveau de pression de consigne à une valeur de pression de consigne supérieure ou égale à 5 hPa, de préférence comprise entre 10 et 90 hPa, lorsque l'information de mode de ventilation correspond à une information de mode de ventilation manuelle.

13. Procédé selon la revendication 10, caractérisé en ce qu'à l'étape (a), on détermine au moins une information du gaz entrant et/ou sortant de l'organe d'accumulation (4) choisie parmi la pression du gaz entrant et/ou sortant de l'organe d'accumulation (4), le débit du gaz entrant et/ou sortant de l'organe d'accumulation (4), la pression du gaz à l'intérieur de l'organe d'accumulation (4) et/ou une information transmise par un transmetteur mécanique ou par un commutateur agencé sur ou à proximité du raccord de l'organe d'accumulation (4).
